# EUROPÄISCHE PATENTANMELDUNG

(11) **EP 2 982 739 A1**
(43) Veröffentlichungstag der Anmeldung: **10.02.2016**
(21) Anmeldenummer: 15002315.8
(22) Anmeldetag: 04.08.2015
(51) Int. Cl.: C12M 1/107, C12M 1/00, C12M 1/06, C12M 1/02

(54) **KOMBINIERTER HYDROLYSE-FERMENTATIONS-APPARAT**

(30) Priorität: 07.08.2014 DE 102014011447
(71) Anmelder: S+B Service und Betrieb GmbH, 86720 Nördlingen (DE)
(72) Erfinder: WERMUTH, Gert, 86609 Donauwörth (DE); STOLLBERG, Bernhard, 08459 Neukirchen (DE); AUERBACH, Hans-Joachim, 08066 Zwickau (DE)
(74) Vertreter: Auerbach, Bettina

(57) **Zusammenfassung**

Die Erfindung betrifft einen kombinierten Hydrolyse-Fermentations-Apparat zur räumlich getrennten Realisierung der Verfahrensstufen Hydrolyse und Methanfermentation in Bioenergieanlagen mittels Nassfermentation. Der Apparat besteht aus wenigstens einer beheizbaren, mit Umwälzeinrichtungen und bedarfsweise belüftbaren Behälter-Kombination, wobei ein korrosionsfest ausgerüsteter Hydrolysebehälter (1) in einem zumindest im Gasraum (20) ebenfalls korrosionsfest ausgerüsteten Behälter für Gärsubstrate (2) zentrisch angeordnet ist, die Wandkonstruktion des Hydrolysebehälters (4) wenigstens teilweise als für Heizwasser durchströmbare Heizfläche (5) ausgebildet ist, der Hydrolysebehälter (1) abgasseitig mit dem Gasraum (7) des umgebenden Behälters für Gärsubstrat (2) in Verbindung steht und für die Belüftung des Hydrolysebehälters (1) Druckluftlanzen (15) und/oder Einrichtungen für die Luftzuführung über die von der Umwälztechnik (11) gebildete Trombe (16) angeordnet sind.

## Beschreibung

Die Erfindung betrifft einen kombinierten Hydrolyse-Fermentations-Apparat für die Verbesserung der stofflichen und energetischen Effizienz von Anlagen zur stofflichen und energetischen Verwertung von Energiepflanzen, von Reststoffen aus der landwirtschaftlichen Pflanzen- und Tierproduktion sowie von biogenen Abfällen aus der Lebensmittel- und Getränkeindustrie. Eine derartige technische Lösung wird für die Gewinnung von Bioenergie mittels Nassfermentation benötigt.

Die Gewinnung von methanhaltigen Brenngasen aus biogenem Material gilt als unverzichtbare Schlüsseltechnologie im Bereich der so genannten Energiegewinnung aus regenerativen Quellen. Sie ist als Multitalent den anderen Techniken zur Nutzung regenerativer Energiequellen insbesondere dadurch überlegen, dass sie
- sowohl für die Versorgung mit elektrischer und thermischer Energie als auch für die Kraftstoffversorgung nutzbar ist,
- mit erprobten und verfügbaren technischen Mitteln speicherbar ist,
- wahlweise der Grundlast- und auch der Spitzenlastversorgung dienen kann,
- mit der Gewinnung besonders pflanzenverfügbarer organischer Stickstoff-Phosphor-Kalium-Schwefel-Düngemittel verbunden werden kann,
- zur Minderung der Emission von Klimaschadgasen beiträgt und
- über zumindest in urbanisierten und/oder industrialisierten Regionen bereits verfügbare und ausreichend leistungsfähige Verteilnetze dezentral versorgungswirksam eingesetzt werden kann.

Es ist bekannt, dass der energetische Ausnutzungsgrad von Energiepflanzen und biogenen Reststoffen mit cellulosischen und lignocellulosischen Anteilen in beachtlichem Maße gesteigert werden kann, wenn diese Stoffe vor ihrem Einsatz in ein anaerobes Milieu einen Zerkleinerungs- und Hydrolyseprozess durchlaufen. Vorgeschlagen wurden ebenfalls bereits technische Lösungen, mit deren Hilfe insbesondere die anaeroben Mindestbehandlungszeiten der Gärsubstrate verlängert werden. Nicht zuletzt sind dem Fachmann verfahrenstechnische und vorrichtungstechnische Lösungen bekannt, mit denen im praktischen Betrieb die energetische Effizienz der Nassfermentation durch den Einsatz von adaptierten mesophilen oder thermophilen Mikrokulturen verbessert werden kann. Dabei besitzt bekanntlich deshalb die Hydrolyse der Einsatzstoffe eine besondere Stellung, weil mit ihr nicht nur die Einsatzstoffe durch die Umwandlung von biogenen Inhaltsstoffen zu kurzkettigen Fettsäuren für den eigentlichen Methanisierungsprozess vorbereitet werden, sondern weil lignocellulosische Strukturen der eingesetzten biogenen Stoffe soweit geschwächt werden können, dass sie zumindest teilweise dem Angriff von Methanbakterien zugänglich sind. Es hat folgerichtig nicht an Versuchen gefehlt, die technischen Lösungen für den Hydrolyseprozess weiterzuentwickeln.

So offenbart die DE 44 09 487 C2 ein Verfahren und eine Anlage zur Vergärung von biogen-organischen Rohabfällen, welches mehrere Fermenter für die getrennte anaerobe Hydrolyse und die anaerobe Fermentation nutzt, wobei die Flüssigphase aus einem im Hydrolysestadium befindlichen Fermenter als Perkolat einem im Methanisierungsstadium befindlichen Fermenter aufgegeben wird und umgekehrt.

Weiterhin wird mit der DE 195 32 359 C2 ein Verfahren zur Biogaserzeugung aus feststoffreicher Biomasse vorgeschlagen, das wenigstens vier Fermenter besitzt, die nacheinander gestartet werden und die sich in unterschiedlichen Prozessstufen befinden. Alle Fermenter sind mit einem Säurepuffer verbunden, aus dem die Fermenter bedarfsgerecht beschickt werden. Als Säurepuffer dient dabei ein Apparat zur Erzeugung von saurem Hydrolysat aus einem Hydrolyseprozess.

Mit der GR 95 100 473 wird eine technische Lösung zur industriellen Verwendung von Müll mit effektiver Wiederverwertung und Optimierung in der Energieerzeugung offenbart.

Dieses Verfahren zur Biogaserzeugung aus feststoffreicher Biomasse sieht eine hydrolytische Vorbehandlung der lignocellulosehaltigen Substrate vor, wobei die Vorbehandlung durch fluidisierendes Einspeisen von Dampf über etwa 12 Stunden bei 80 bis 90 °C erfolgt, wodurch sich der anaerobe Abbau von 50 bis 55 % auf 80 bis 85 % verbessern soll.

Eine verbesserte technische Lösung enthält die US 2002/0102673 A1, wonach lignocellulosereiche Einsatzstoffe batchweise in wenigstens zwei parallel betriebenen Hydrolyseapparaten anaerob vorbehandelt werden. Anschließend gelangen die erzeugten Hydrolysate in einen Methanbakterien enthaltenden Fermenter, wobei das Gärsubstrat aus dem Methanfermenter zum Perkolieren der Einsatzstoffe in den Hydrolyseapparaten eingesetzt wird.

Ein Verfahren und Vorrichtungen zur kontinuierlichen Verarbeitung erneuerbarer Rohstoffe werden mit der EP 1836 181 B1 bekannt gemacht. Dabei dient die hydrolytische Vorbehandlung von lignocellulosischen Einsatzstoffen mittels Druckhydrolyse bevorzugt der Gewinnung von Zuckerlösungen für die anschließende Hefegärung zur Ethanolgewinnung.

Mit der DE 2004 053 615 B3 wird ein Abbauverfahren von biogenem Material beschrieben. Die dort entwickelte technische Lösung dient der bedarfsgerechten Steuerung der Biogaserzeugung. Dazu werden mehrere aerob betriebene Perkolatoren betrieben, denen die Flüssigphase entzogen, in einem Pufferbehälter zwischengestapelt und anschließend einem oder mehreren Methanfermentern zur Gewinnung von Biogas zugeführt wird.

Ein Verfahren zum Betreiben einer Feststofffermenteranlage sowie eine Vorrichtung hierzu beschreibt die DE 10 2005 029 306 B4.

Enthalten ist der Vorschlag zur zeitlich versetzten Prozessführung in wenigstens zwei batchweise betriebenen Fermentationsbereichen für trockensubstanzreiche und perkolierbare Substrate, wobei mit den Perkolaten des jeweils jüngeren Produktes bei pH-Werten von etwa 2,5 die gebildeten Säuren ausgeschwemmt werden und dem älteren Produkt zur Methanisierung zugefügt werden.

Das mit der DE 10 2006 006 743 A1 offenbarte Verfahren zur energetischen Nutzung von organisch belasteten Abwässern durch Erzeugung von Biogas enthält auch einen Hydrolyseschritt.

Die vorgeschlagene Hydrolyse besteht in einer der dort beschriebenen Varianten aus zwei dem eigentlichen Methanfermenter vorgeschalteten Hydrolyseapparaten, die allerdings in Reihe geschaltet sind.

Sie sind außerdem als Festbettreaktoren ausgebildet, so dass sie in erster Linie für die fermentative Abwasserbehandlung in Frage kommen. Auf die vorteilhafte Betriebsweise der hydrolytischen Behandlung im aeroben Milieu wird hier bereits hingewiesen.

Im "Forum der Forschung BTU Cottbus, 19/2006" wird ein zweistufiges Fest-Flüssig-Biogasverfahren mit offener Hydrolyse als ein neues technologisches Konzept für die Biogasgewinnung aus nachwachsenden Rohstoffen und bioverfügbaren Abfällen beschrieben. Dargestellt werden Untersuchungsergebnisse zu der einer Fermentationsstufe vorgeschalteten aeroben oder schwach aeroben Hydrolyse als technologisches Konzept für die Biogasgewinnung. Der Vorschlag enthält auch die Rückführung von so genannter Perkolatflüssigkeit in die Hydrolysestufe, allerdings ohne vorherige Hemmstoffentfrachtung. Zudem werden die Hydrolysegase mittels der offenen Hydrolyse in die Atmosphäre entlassen, weil sie überwiegend kohlendioxidhaltig seien. Das widerspricht praktischen Erfahrungen ebenso, wie anderen Veröffentlichungen, die auf den Gehalt an Wasserstoff und Fettsäuredämpfen in den Hydrolysegasen hinweisen. Die Auswirkungen der vorgeschalteten aeroben Hydrolyse auf den Fermentationsprozess werden sowohl in der beschleunigten Methanfermentation (16-20 Tage für Maissilage und 5-7 Tage für Bioabfälle) und in der höheren Methanausbeute gesehen.

Der Vorschlag für eine Biogasanlage und für ein Verfahren zur Erzeugung von Biogas kann der DE 10 2008 015 609 A1 entnommen werden. Auch dieser Vorschlag bezieht sich auf den räumlich von dem Methanbildungsprozess getrennten vorgeschalteten Hydrolyseprozess. Es werden wenigstens zwei Hydrolysebehälter vorgesehen, die batchweise betrieben werden sollen. Die Hydrolysetemperatur soll zwischen 30 und 60 °C gewählt werden, wobei von einer mindestens zwei Tage dauernden Hydrolysebehandlung ausgegangen wird. Von einer aeroben hydrolytischen Behandlung wird ebenso wenig gesprochen, wie von der Nutzung der Hydrolysegase und von der Vorbehandlung der flüssigen Gärreste zum Anmaischen der zu hydrolysierenden Einsatzstoffe.

Aus der Vortragsammlung der Fachtagung Biogas 2008 sind veröffentlichte Untersuchungsergebnisse zur Hydrolyse bei Biogasanlagen bekannt. Dort werden die Anforderungen an die Stufen im Biogasprozess benannt. Die Auswirkungen einer dem Fermentationsprozess vorgeschalteten anaeroben oder aeroben Hydrolyse werden quantifiziert dargestellt. Vorgeschlagen wird demnach eine einstufige hydrolytische anaerobe Hydrolyse bei 25 bis 35 °C zur Erhöhung der Methanausbeute. Mit der DE 10 2010 010 091 A1 wird das so genannte Bioliquid-Verfahren bekannt gemacht. Das vorgeschlagene Verfahren sieht die Abscheidung von Störstoffen aus den verfügbaren Einsatzstoffen in einem belüfteten Vorbehälter vor. Unabhängig von einem zwischengeschalteten thermomechanischen Aufschluss überwiegend lignocellulosehaltiger Einsatzstoffe soll das Einsatzstoffgemisch anschließend zunächst aerob vorbehandelt und danach einem Hydrolyseschritt unterworfen werden. Das gewonnene Hydrolysat wird in eine feste und in eine Flüssige Phase getrennt. Während die feste Phase in die aerobe Vorbehandlung (hier Rotte genannt) zurückgeführt wird, gelangt die flüssige Phase in den Anaerobfermenter zur Biogasgewinnung.

Das "Bayern Biogas Forum", Nr. III - 3/2010 veröffentlich das "Prozessmodell Biogas". Das beschriebene Modell sieht die hydrolytische Vorbehandlung von lignocellulosereichen Einsatzstoffen für die Biogasgewinnung vor.

Es wird auf die positiven Wirkungen der Einsatzstoffzerkleinerung und der Enzymzugabe hingewiesen. Angaben zur batchweisen Behandlung, zur Bewertung der Wirkung eines aeroben Milieus oder zur bevorzugten Behandlungstemperatur können den Veröffentlichungen allerdings nicht entnommen werden.

Das "Haus der Bayerischen Landwirtschaft" vom 06.12.2010 empfiehlt, mit einem Hydrolysefermenter Gras für die Biogasanlage fit zu machen. Dabei wird auf die Wirkungen von Hydrolyse und Versauerung von Biosuspensionen vor dem Methanbildungsprozess hingewiesen.

Wegen der Bildung von Wasserstoff und Kohlendioxid beim Hydrolyseprozess sei dieser mit einem Energieverlust verbunden, der allerdings durch einen Energiegewinn infolge des intensiveren biologischen Aufschlusses kompensiert würde. Hingewiesen wird auf die zusätzlichen Wirkungen eines vorgeschalteten Hydrolyseschritts in Form
- eines besseren biologischen Aufschlusses der Einsatzstoffe,
- der Beschleunigung des Kohlenstoffabbaus,
- der Herabsetzung der Viskosität des Hydrolysats,
- der Vermeidung der Übersäuerung des Anaerob-Fermenters,
- der Schwimmschichtreduzierung,
- des geringerer Rührenergiebedarfs und
- der stabileren Methanisierungsphase.

Das mit der DE 10 2011 018 912 A1 bekannt gemachte Verfahren zur Biogasherstellung und die dafür beschriebene Biogasanlage unterscheidet zwischen einer Vollstrom- oder einer Teilstromhydrolyse. Es bezeichnet den Hydrolyseschritt sowohl als kontinuierlichen als auch als einen Batchprozess. In allen Fällen ist der Hydrolyseschritt jedoch zwischen einem vorgeschalteten und einem nachgeschalteten Methanfermentationsprozess angeordnet.

Während der 8. Biogasfachtagung: Biogas aus festen Abfällen und Reststoffen 09/2011 wird über Wasserstoffperoxid als Oxidationsmittel während der zwischengeschalteten aeroben Hydrolyse informiert. Der bekannt gemachte Vorschlag sieht in einem ersten Vergärungsschritt das anaerobe Umsetzen der leicht abbaubaren Verbindungen der verfügbaren Einsatzstoffe vor. In einem zweiten Bearbeitungsschritt soll das Gärsubstrat mit überwiegend schwer abbaubaren Verbindungen unter Einsatz von Sauerstoff in einem Hydrolysereaktor begast werden. Das gewonnene Hydrolysat kann anschließend erneut dem vorgeschalteten oder einem nachgeschalteten Anaerob-Fermenter zugeführt werden.

Ein in der DE 10 2012 008 518 A1 offenbarte Verfahren zum Erzeugen von Biogas aus Biomasse mit Hilfe eines methanogenen Mikroorganismus sieht die stetige Zugabe wenigstens eines adaptierten Mikroorganismus (Impfmasse) zum Gärsubstrat vor.

Die EBT Energie- & Biotechnik GmbH (Firmenschrift von 09/2013) benennt neue Herausforderungen durch alternative Substrate. Dabei wird auf die Möglichkeiten der Biogasgewinnung aus Stroh, Mist und Landschaftspflegematerial hingewiesen. Dazu soll bevorzugt eine Kombination aus mechanischer und biologischer Vorbehandlung dieser Materialien angewendet werden. Wegen der Biologie als der "größten Kraft" soll generell eine Hydrolyse-Vorstufe und bei schwierigen Subtraten eine mechanische Zerkleinerung, im dargestellten Beispiel mittels Querstromzerspaner, erwogen werden. Angaben zur Gestaltung des Hydrolyseschrittes werden nicht gemacht.

Die bionova GmbH publiziert in einer Firmenschrift neuere Ergebnisse der Untersuchungen zur Hydrolyse in Biogasprozessen mit nachwachsenden Rohstoffen. Die Ergebnisse aus einer Vergleichsbewertung von aerober und anaerober Hydrolyse nach einer 48 - stündigen Behandlungszeit werden dargestellt.

Unterschieden wird dabei auch zwischen einer kurzzeitigen (4 Stunden) und einer Langzeit- (48 Stunden) Hydrolyse sowie zwischen kontinuierlicher und batchweiser hydrolytischen Behandlung.

Die avantec biogas GmbH berichtet in der Firmeninformation über die so genannte Avantec-Hydrolyse. Sie wird als eigenständige vorgeschaltete biologische Prozessstufe vor einer Methanfermentation dargestellt. Während die konkurrierende anaerobe Hydrolyse zwischen 5 und 10 Tagen Behandlungszeit erfordern würde, dafür jedoch ein Gasgemisch aus 50 % Wasserstoff und 50 % Kohlendoxid liefern würde, soll die aerobe Avantec-Hydrolyse kein Methan und auch keinen Wasserstoff freisetzen, nach wenigen Stunden abgeschlossen sein und durch eine starke Geruchsreduzierung infolge der verstärkten Essigsäurebildung gekennzeichnet sein.

Den bisher bekannt gemachten technischen Lösungen haftet der gemeinsame Mangel an, dass praktikable Anleitungen für die apparative Gestaltung der Hydrolysestufe in Verbindung mit einer leistungsfähigen Methanfermentationsanlage nicht aufgefunden werden können. Oft wird die offensichtliche Vorteilshaftigkeit
- einer räumlich vom eigentlichen Methanisierungsprozess getrennten Hydrolysestufe,
- der hydrolytischen Behandlung im aeroben oder im anaeroben Milieu,
- der zu bevorzugenden Prozesstemperaturen sowie
- der Nutzung der zu erwartenden Unterschieden zwischen kontinuierlicher und batchweiser Prozessführung betont. Allerdings geschieht dies ohne das Benennen geeigneter Vorschläge für das Anwenden solcher verfahrenstechnischer Erkenntnisse in neugestalteten und gegebenenfalls auch für das Nachrüsten bestehender und insbesondere in der Landwirtschaft verbreiteter Anlagen zur Nassfermentation biogener Roh- und Abfallstoffe.

Die Aufgabe der Erfindung besteht deshalb in der Überwindung der Mängel des bekannten Standes der Technik. Insbesondere soll eine technische Lösung entwickelt werden, mit deren Hilfe die theoretisch fundierten Ergebnisse energie- und kostengünstig sowohl für neue als auch für bestehende Bioenergieanlagen auf der Grundlage der Methanfermentation mittels Durchfluss-Rührbehältern angewendet werden können.

Die Aufgabe der Erfindung wird durch die Merkmale des Anspruchs 1 gelöst. Vorteilhafte Ausgestaltungen der Erfindung werden mit den Unteransprüchen gekennzeichnet.

Danach besteht ein Kombinierter Hydrolyse-Fermentations-Apparat zur räumlich getrennten Realisierung der Verfahrensstufen Hydrolyse und Methanfermentation in Bioenergieanlagen mittels Nassfermentation aus wenigstens einer beheizbaren, mit Umwälzeinrichtungen und bedarfsweise teilweise belüftbaren Behälter-Kombination.

Die erfindungsgemäße Ausführung dieser Apparate-Kombination sieht vor, dass ein korrosionsfest ausgerüsteter Hydrolysebehälter in einem ebenfalls wenigstens teilweise korrosionsfest ausgerüsteten Behälter für Gärsubstrate zentrisch angeordnet ist. Während die äußere Wandkonstruktion und gegebenenfalls auch eine feste Deckenkonstruktion aus wasserdichtem Stahlbeton zumindest im Bereich des Gasraumes durch polymere Anstriche oder Werkstoffe korrosionsfest ausgerüstet sind, soll der Hydrolysebehälter zumindest eine Wandkonstruktion aus korrosionsbeständigem Stahlblech besitzen. Die Wandkonstruktion des Hydrolysebehälters ist darüber hinaus wenigstens teilweise als für Heizwasser durchströmbare Heizfläche ausgebildet. Weiterhin ist vorgesehen, dass der Hydrolysebehälter abgasseitig mit dem Gasraum des umgebenden Behälters für Gärsubstrat in Verbindung steht und dass für die Belüftung des Hydrolysebehälters Druckluftlanzen und/oder Einrichtungen für die Luftzuführung über die von der Umwälztechnik gebildete Trombe angeordnet sind. Für die Gewährleistung der Apparatefunktion für die aerobe Hydrolysierung ist es auch möglich, anstelle von Einrichtungen für die Luftzuführung solche für die Sauerstoffzuführung vorzusehen.

In einer bevorzugten Ausführungsvariante steht der Hydrolysebehälter mit Einrichtungen für die dosierte Luftzuführung in Verbindung.

Damit lässt sich die Hydrolyse sowohl im schwach aeroben oder im aeroben Milieu realisieren. Die Luft- oder Sauerstoffzuführung kann dabei in komprimierter Form über Luftlanzen im Behältertiefsten und/oder mittels Gebläsen in den Gasraum des Hydrolysebehälters erfolgen, von wo aus das sauerstoffhaltige Hydrolysegas wiederholt über die vom Zentralrührwerk des Hydrolysebehälters gebildete Trombe mit der suspendierten Biomasse in Kontakt gebracht wird.

In einer anderen Lösungsvariante ist die Heizfläche des Hydrolysebehälters derart dimensioniert, dass eine die maximale Medientemperatur im Hydrolysebehälter von bis zu 60 °C erreicht wird. Gleichzeitig ist der den Hydrolysebehälter umgebende Fermentationsbehälter für Gärsubstrat ohne spezielle Heizflächen ausgebildet. Dies führt nicht nur zur Vereinfachung und kostengünstigeren Ausbildung der Fermenterkonstruktion, sondern erleichtert gleichzeitig die Umwälzung des Gärsubstrates bei Einsatz deutlich geringer belasteter Rührmechanismen.

Eine weitere vorteilhafte Ausführung des Kombinierten Hydrolyse-Fermentations-Apparates sieht vor, die senkrechten Umfassungswände des Hydrolysebehälters thermisch unisoliert auszubilden. Gleichzeitig fungieren die senkrechten Umfassungswände des Hydrolysebehälters als Heizflächen des umgebenden Behälters für das Gärsubstrat, ohne damit das Umwälzen des Gärsubstrates zu behindern oder Ursache für Verunreinigungen der Heizflächen zu sein.

Zur Vermeidung von Umweltbelastungen infolge des Entweichens von Gärgasen aus den Fermentationsbehältern müssen diese gasdicht abgedeckt sein.

Dazu werden solche Reaktionsgefäße trotz der teilweise beachtlichen Behälterabmessungen mit festen oder flexiblen Abdeckungen ausgestattet. In einer besonderen Ausführungsvariante des Kombinierten Hydrolyse-Fermentations-Apparates fungiert der Hydrolysebehälter deshalb als Stütze für die gasdichte Abdeckung des Behälters für Gärsubstrat. Dabei stellt der Hydrolysebehälter die Mittelstütze für die Aufnahme des Gurt- oder Balkensystems zur Ablage der Gasspeicherfolie dar. Gleichzeitig kann er auch für die Aufnahme der erhöht angeordneten Gasentnahmeleitung dienen. Im Falle der festen Abdeckung des Fermentationsbehälters mittels korrosionsfest ausgestatteter Metallkonstruktion oder einer wenigstens einseitig beschichteten Stahlbetonplatte ermöglicht der Hydrolysebehälter als Stütze eine deutlich vereinfachte bauliche Gestaltung der Behälterabdeckung.

Es ist außerdem möglich, den in einem Kombinierten-Hydrolyse-Fermentations-Apparat den Hydrolysebehälter umgebenden Raum für Gärsubstrat als Fermenter, als Nachfermenter oder als Endlager für Gärreste zu nutzen. Damit ist es auf vergleichsweise einfache Weise möglich, in einer bestehenden Anlage für die energetische Verwertung von Biomasse mittels Nassfermentation einen noch nicht gasdicht abgedecktes Endlager-Behälter mit einem Hydrolysebehälter als Mittelstütze für eine nachträgliche gasdichte Abdeckung und als Heizung für die verlängerte Aufrechterhaltung des Fermentationsprozesses zu nutzen.

Eine besondere Bedeutung besitzt eine mögliche Ausführungsvariante des Kombinierten-Hydrolyse-Fermentations-Apparates, gemäß der wenigstens zwei Kombinierte-Hydrolyse-Fermentations-Apparate für den wechselseitigen Batchbetrieb des Hydrolysebehälters und/oder des Behälters für Gärsubstrat in Verbindung stehen. Mit einer solchen Ausführung wird der entscheidende verfahrenstechnische Funktionsmangel von üblichen Durchfluss-Rührfermentern für die Nassfermentation in Anlagen für die energetische Verwertung von Biomasse kompensiert, weil die Mindestbehandlungszeit im anaeroben Milieu von üblicherweise weniger als einem Tag auf wenigstens die Behandlungszeit im Batchbetrieb verlängert wird.

In einer weiteren bevorzugten Ausführungsvariante des Kombinierten-Hydrolyse-Fermentations-Apparates ist der Hydolysebehälter und/oder der umgebende Behälter für Gäsubstrat mit den Wechsel der Umwälztechnik ohne Betriebsunterbrechungen sichernden Tauchhülsen ausgestattet. In einer sehr einfachen Form gelingt dies bei Wahl einer festen gasdichten Abdeckung des den Hydrolysebehälter umgebenden Fermenters. In diesem Fall sind die Montageöffnungen für die Mechanismen zur Umwälzung der Biosuspension im Hydrolysebehältzer und/oder des Gärsubstrates im umgebenden Fermenter mit Tauchhülsen ausgestattet, mit deren Hilfe bei zielgerichteter geringfügiger Überfüllung der jeweiligen Reaktionsräume über die Nennfüllstände hinaus die Gasdichtigkeit der Reaktionsräume aufrechterhalten und erforderliche Manipulationen an den Rührmechanismen ohne Personalgefährdung und praktisch ohne Funktionsbeeinträchtigungen ermöglicht werden.

Es hat sich als ausreichend erwiesen, wenn der Hydrolysebehälter in einem Kombinierten-Hydrolyse-Fermentations-Apparat solche Dimensionen erhält, mit denen das Nutzvolumen des Hydrolysebehälters wenigstens das doppelte Volumen der täglichen Dosismenge für die Bioenergieanlage beträgt.

Insbesondere beim batchweisen Betrieb von wenigstens zwei derartigen Apparate-Kombinationen ist damit die wenigstens zweitägige hydrolytische Behandlung der eingesetzten Biosuspensionen sicher gewährleistet.

In einer weiteren bevorzugten Ausführungsvariante der Erfindung ist der Kombinierte-Hydrolyse-Fermentations-Apparat mit einem separaten und räumlich von der Fermentation getrennten Entschwefelungsapparat gekoppelt. In diesem Fall gelangen die sauerstoffhaltigen Abgase aus dem Hydrolysebehälter nicht nur in den umgebenden Fermentationsraum, sondern anschließend auch in die zusätzliche Entschwefelungsstation. Für die dort in einem üblicherweise eingesetzten Rieselkörperapparat tätigen Schwefelbakterien bildet der im zugeführten Biogas enthaltene Sauerstoff wenigstens anteilig das die Einbindung des Schwefelwasserstoffs in nicht flüchtige Schwefelverbindungen sichernde Reaktionsmittel.

Die Vorteile der erfindungsgemäßen technischen Lösung gegenüber dem Stand der Technik lassen sich wie folgt zusammenfassen:
- Da der Hydrolysebehälter bevorzugt mit einer Medientemperatur von bis zu 60 °C betrieben wird, stellt er gleichzeitig die Heizfläche für den umgebenden Fermenter mit wählbaren Medientemperaturen für ein mesophiles (32 bis 42 °C) oder thermophiles (45 bis 55 °C) Milieu dar, wobei der umgebende Fermenter eine zusätzliche Heizflächenausstattung nicht benötigt.
- Wegen der Dimensionierung des Hydrolysebehälters auf ein Nutzvolumen von zwei bis drei der Fermentationsanlage zuzuführenden Tagesrationen wird der Reaktionsraum des umgebenden Fermenters praktisch kaum beeinträchtigt.
- Unabhängig davon, ob der umgebende Fermenter für die Haupt- oder Nachfermentation genutzt wird, ist mit vergleichsweise einfachen technischen Mitteln eine vollständige Umwälzung des Fermentationsmediums gewährleistet, ohne mögliche Totzonen und gegebenenfalls auch Sedimentablagerungen im Zentrum des Fermenters in Kauf nehmen zu müssen.
- Durch die Nebenfunktion des Hydrolysebehälters als Heizeinrichtung für den umgebenden Fermenter benötigt der Hydrolysebehälter weder eine Außenwandisolierung noch eine Witterungsschutzverkleidung.
- Die sauerstoffhaltigen Hydrolysegase gelangen auf einfachste Weise in den umgebenden anaeroben Reaktionsraum, in dem nicht nur unter Nutzung des eingetragenen Sauerstoffs die weitgehende Einbindung des im Fermenter entbundenen gasförmigen Schwefelwasserstoffs als nicht flüchtige Schwefelverbindungen in das Gärsubstrat erfolgt, sondern auch die im Hydrolysegas mitgeführten Anteile an Wasserstoff und Dämpfen kurzkettiger Fettsäuren zur Qualitätsverbesserung des im Fermenter verfügbaren Biogases beitragen.
- Durch die flächige Belastung der Bodenkonstruktion des Fermenters kann auf die für übliche Mittelstützen erforderliche Verstärkung der Bodenkonstruktion verzichtet werden.
- Sowohl für starre als auch für flexible Abdeckungen des Fermenters vereinfachen sich sie Anforderungen für die Ausführung einer festen Deckenkonstruktion ebenso, wie für die Ablage einer flexiblen Gasspeichermembran.
- Der Kombinierte-Hydrolyse-Fermentations-Apparat ist bei wenigstens zweifacher Anordnung in einer Anlage zur energetischen Verwertung von Biomasse mittels Nassfermentation sowohl für das batchweise Hydrolysieren als auch für das batchweise Fermentieren geeignet.
- Der Kombinierte-Hydrolyse-Fermentations-Apparat ist bei wenigstens zweifacher Anordnung in einer Anlage zur energetischen Verwertung von Biomasse mittels Nassfermentation auch zur wirksamen Hygienisierung durch das wenigstens 10-tägige batchweise Fermentieren im thermophilen Milieu geeignet.

Die Erfindung soll nachstehend mit Ausführungsbeispielen näher erläutert werden.

In der beigefügten Zeichnung zeigen
- Fig. 1: die schematische Schnittdarstellung eines Kombinierten-Hydrolyse-Fermentations-Apparates, bestehend aus zwei ineinander angeordneten zylindrischen Behältern mit gasdichter Abdeckung durch Metallkonstruktionen;
- Fig. 2: die schematische Schnittdarstellung eines Kombinierten-Hydrolyse-Fermentations-Apparates, bestehend aus zwei ineinander angeordneten zylindrischen Behältern mit gasdichter Abdeckung des umgebenden Fermenters mit einer Stahlbetonplatte;
- Fig. 3: die schematische Schnittdarstellung eines Kombinierten-Hydrolyse-Fermentations-Apparates, bestehend aus zwei ineinander angeordneten zylindrischen Behältern mit gasdichter Abdeckung des umgebenden Fermenters durch einen Doppelmembrangasspeicher.

### Ausführungsbeispiel 1:

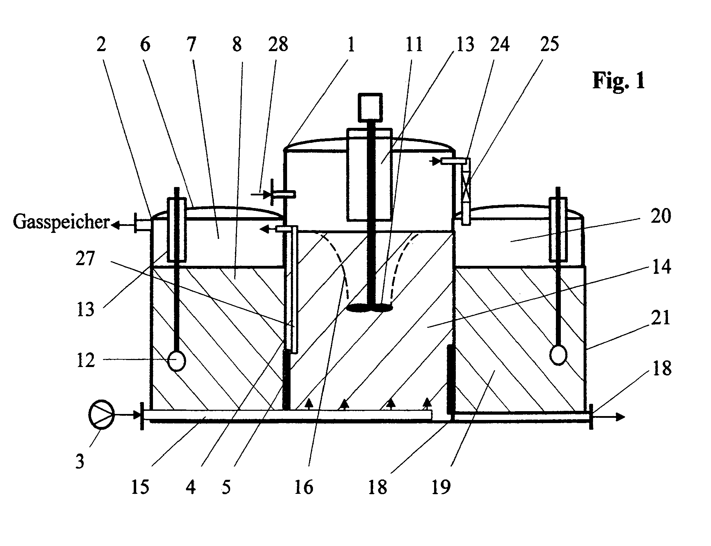

In einer Anlage für die energetische Verwertung von lignocellulosereicher Biomasse in Form von 35 % Rindermist und 65 % Rapsstroh, bezogen auf die Menge der eingesetzten Originalsubstanz, mittels Nassfermentation wird die aus den zerkleinerten Einsatzstoffen und dem aus der Phasentrennung der Gärreste gewonnenen Biofiltraten hergestellte Biosuspension gemäß der Fig. 1 dem Hydrolysebehälter 1 eines Kombinierten-Hydrolyse-Fermentationsapparates zugeführt.

Das nutzbare Volumen 14 für die hydrolytische Behandlung im Hydrolysebehälter 1 beträgt 200 m³. Der komplett aus Edelstahl gefertigte zylindrische Hydrolysebehälter 1 hat einen Durchmesser von 6,5 m, eine Gesamthöhe von 10 m und ein lichtes Volumen von 332 m³. Er besitzt einen angeschweißten Flachboden und weist im Tiefsten einen Auslass 18 für eventuell auftretende Sinkschlämme auf. Der den Hydrolysebehälter 1 umgebende Fermenterbereich 8 besitzt eine kreisringförmige Grundfläche mit einen lichten äußeren Durchmesser von 30 m, eine Höhe von ebenfalls 8 m und damit ein Füllvolumen von 5.386 m³, das bis zur Füllhöhe von 7 m mit einem Nutzvolumen 19 von 4.713 m³ für die anaerobe Behandlung des im Hydrolysebehälter 1 hergestellten Hydrolysats zur Verfügung steht. Sowohl der Hydrolysebehälter 1 als auch der diesen umgebenden Fermenter 8 sind mit leicht gewölbten Blechkonstruktionen aus korrosionsbeständigem Werkstoff gasdicht abgedeckt, sind dadurch begehbar und auch bei begrenzten Über- und Unterdrücken in den Gasräumen 20 der verbundenen Reaktionsbehälter standsicher.

Der Hydrolysebehälter 1 ist mit einem Zentralrührwerk 11 ausgestattet, dessen Rührflügel etwa 6 m in den Hydrolysebehälter 1 hineinragt. An der unteren Innenwand des Hydrolysebehälters 1 sind gebogene Walzprofile aus Edelstahl für die Aufnahme des Heizmediums in Form von Heizwasser mit einer Temperatur von bis zu 90 °C angeordnet, die zugleich die Behälterwand 4 des Hydrolysebehälters 1 für die Fälle stabilisieren, in denen der Sonderlastfall "entleerter Fermenter und gefüllter Hydrolysebehälter" oder der Sonderlastfall " gefüllter Fermenter und entleerter Hydrolysebehälter" auftreten.

Die planmäßige Nutzung der Behälterkombination sieht vorliegenden Ausführungsbeispiel die weitgehende Aufrechterhaltung der Behälter-Nennfüllstände vor. Wegen der durch den Betrieb des Zentralrührwerks 11 bewirkten Turbulenz der Biosuspension im Hydrolysebehälter 1 wird die eingetragene Heizenergie vom Heizregister 5 im unteren Bereich des Hydrolysebehälters 1 auf die gesamte bespülte zylindrische Wand 4 des Hydrolysebehälters 1 gleichmäßig verteilt, so dass nahezu die gesamte Kontaktwand des Hydrolysebehälters 4 mit dem umgebenden Fermenter 8 für diesen als Heizfläche 5 zur Verfügung steht. Ohnehin hat diese Heizfläche 5 nur die fühlbare Wärme des aus dem Gasraum 7 des Fermenters 8 abgeleiteten Biogases, des bei der Dosierung von Hydrolysat in den Fermenter 8 in den Nachfermenter 9 oder in das Endlager 10 verdrängten Gärrestes sowie die über die Außenwände des Fermenters 21 trotz vollständiger Wärmeisolierung unvermeidlichen Abstrahlungsverluste auszugleichen. Der Gasraum 7 des Fermenters 8 steht mit einem Doppelmembrangassspeicher 22 in Verbindung.

Die luftgestützte Witterungsschutzmembran 23 dieses Gasspeichers 22 bewirkt einen mittleren Arbeitsdruck des gespeicherten Gases in Höhe von 4 mbar. Gegen diesen Speicherdruck fließt das erzeugte Biogas aus dem Fermenter 8 ab. Das sauerstoffhaltige Hydrolysegas 17 gelangt mittels einer oberhalb der Behälterabdeckung 6 angeordneten Überströmleitung 24, die mit einer Rückschlagklappe 25 ausgestattet ist, in den Gasraum 7 des Fermenters 8. Der in den Fermenter 8 mit dem Hydrolysegas eingetragene Sauerstoff dient den auf der Oberfläche des Gäsubstrates und an der Innenfläche des Gasraumes 7 des Fermenters 8 angesiedelten Schwefelbakterien als Reaktionsmittel bei der Umwandlung des im Biogas enthaltenen Schwefelwasserstoffs in nichtflüchtige Schwefelverbindungen, die mit dem Gärrest aus dem Fermenter 8 ausgetragen werden.

Die ebenfalls in Abhängigkeit von den Hydrolysebedingungen im Hydrolysegas enthaltenen Anteile an Wasserstoff, Kohlendioxid und Dämpfen kurzkettiger Fettsäuren dienen im anaeroben Milieu des den Hydrolysebehälter 1 umgebenden Fermenters 8 der zusätzlichen Bildung von energiereichem Biogas.

Die regelmäßige Umwälzung des Gärsubstrates im Fermenter 8 erfolgt durch drei höhenverstellbare Tauchmotor-Rührwerke 12, die von der oberen Abdeckung 6 des Fermenters 8 über an sich bekannte Service-Plattformen 26 bedient werden. Sowohl am Zentralrührwerk 11 des Hydrolysebehälters 1 als auch an den Tauchrührwerken 12 des Fermenters 8 sind Tauchhülsen 13 angeordnet, deren untere Enden etwa 10 cm über der im Regelbetrieb erreichten maximalen Füllstandshöhe im jeweiligen Behälter angeordnet sind.

Im Wartungs- und Reparaturfall wird die jeweilige Füllstandshöhe um wenigstens 15 cm überschritten, wodurch eine gefährdungsfreie Arbeit an den jeweiligen Rührmechanismen 11, 12 ermöglicht wird.

Täglich werden dem Hydrolysebehälter 1 etwa 80 m³ Biosuspension mit einem durchschnittlichen Trockensubstanzgehalt zwischen 18 und 20 % von oben über die Biosuspensionszuführung 28 aufgegeben. Dabei werden aus dem unteren Bereich des Hydrolysebehälters 1 Hydrolysatmengen mit etwa dem Volumen der zugeführten Biosuspension über eine Steigrohrleitung 27 mit einer freien Querschnittsfläche von 300 cm² in den umgebenden Fermenter 8 ausgetragen, wobei die unausreichend hydrolysierte und Schwimmschichten bildende Biomasse deutlich länger als die mittlere hydrolytische Behandlungszeit im Hydrolysebehälter 1 verbleibt.

Aus dem Fermenter des Kombinierten-Hydrolyse-Fermenter-Apparates wird Gärrest mit einem mittleren Trockensubstanzgehalt von 10 bis 12 % und Biogas mit einer Feuerungswärmeleistung von 2.500 kW ausgetragen.

### Ausführungsbeispiel 2:

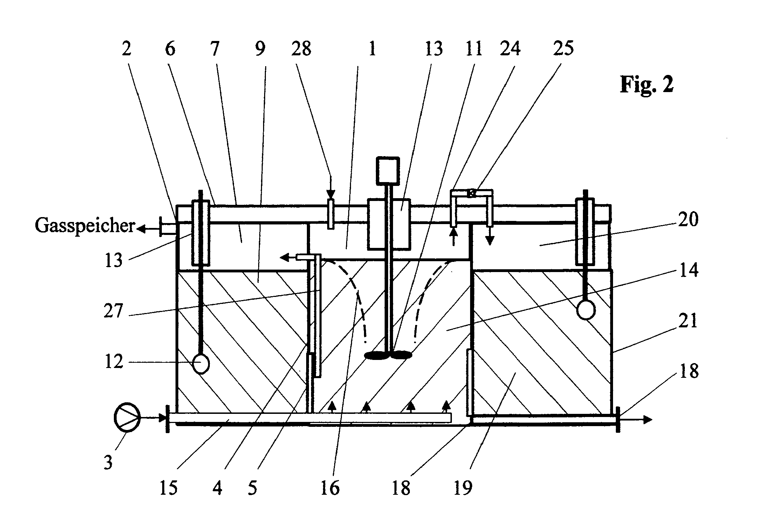

In einer Anlage für die energetische Verwertung von lignocellulosereicher Biomasse in Form von 70 % Hühnermist und 30 % Maisstrohsilage, bezogen auf die Mengen der eingesetzten Originalsubstanz, mittels Nassfermentation wird die aus den zerkleinerten Einsatzstoffen und dem aus der Phasentrennung der Gärreste gewonnenen Biofiltraten hergestellte Biosuspension gemäß der Fig. 2 dem Hydrolysebehälter 1 eines Kombinierten-Hydrolyse-Fermentations-Apparates, der in der Anlage für die energetische Verwertung von Biomasse mit einer Feuerungswärmeleistung von 1,3 MW in zweifacher Ausführung zur Verfügung steht, zugeführt. Der zylinderförmig ausgebildete Hydrolyebehälter 1 ist jeweils zentral in einem zylinderförmig ausgebildeten Rührfermenter aus Stahlbeton angeordnet. Die als Nachfermenter 9 genutzten Betonbehälter verfügen über einen lichten Außendurchmesser von 28 m und eine lichte Höhe von 6 m.

Die darin angeordneten Hydrolysebehälter 1 sind geschlossene Behälter aus Edelstahlblech mit einem Durchmesser von 4,8 m und einer Höhe von ebenfalls 6 m, wobei bei einem Nennfüllstand des Hydrolysebehälters 1 von 4,5 m jeweils etwa 80 m³ Nutzvolumen 14 zur Verfügung stehen.

Die gesamte Behälterkombination ist mit einer gemeinsamen Stahlbetonplatte gasdicht abgedeckt. Der umgebende Reaktionsraum wird für das batchweise Fermentieren genutzt.

Er wird im Regelbetrieb bis zu einem maximalen Füllstand von 5,5 m genutzt, wodurch im Regelbetrieb bis zu 3.285 m³ Nutzvolumen 19 zur Verfügung stehen.

Der Hydrolysebehälter 1 ist wie im Beispiel 1 ausgestattet, und besitzt ein Zentralrührwerk 11, dessen Rührflügel lediglich 2 m in die zu hydrolysierende Biosuspension eintaucht. Im Gegensatz zum Beispiel 1 werden die Behälter der beiden installierten Kombinierten- Hydrolyse-Fermentations-Apparate sowohl für das batchweise Hydrolysieren der eingesetzten Biosuspensionen bei bis zu 60 ° als auch für das batchweise Fermentieren der Hydrolysate aus den Hydrolysebehältern 1 genutzt.

Auf diese Weise können den Hydrolysebehältern 1 Hydrolysate entnommen werden, die wenigstens einer 48-stündigen aeroben hydrolytischen Behandlung unterworfen wurden.

Durch den erforderlichen Einsatz von täglich etwa 18 t eines Gemischs aus Hähnchenmist und Maisstrohsilage und etwa 20 t aus dem anfallenden Gärrest gewonnenem Biofiltrat werden mit Hilfe der batchweisen Vorhydrolyse, der anschließenden anaeroben batchweisen Fermentation im thermophilen Milieu Gärreste gewonnen.

Diese Gärreste durchlaufen bei einer wenigstens 30 %-igen Teilentleerung des batchweise und vollständig gefüllt betriebenen Fermenters 9 bis zur Wiederbefüllung des zweiten Kombinierten-Hydrolyse- Fermentations-Apparates mit täglich maximal 35 m³ Hydrolysat aus dem Hydrolysebehälter 1 eine garantierte Mindestbehandlungszeit im thermophilen Milieu von wenigstens 960 m³ : 35 m³/d = 27,4 Tagen. Die aus diesen Gärresten gewonnenen Presskuchen mit etwa 30 % TS sind vollständig hygienisiert und weisen eine Konzentration an den wichtigsten Pflanzennährstoffen in Höhe von wenigstens 70 kg/t auf.

### Ausführungsbeispiel 3:

In einer Anlage für die energetische Verwertung von lignocellulosereicher Biomasse in Form von 100 % Rapsstroh mittels Nassfermentation wird die aus den zerkleinerten Einsatzstoffen und den aus der Phasentrennung der Gärreste gewonnenen Biofiltraten hergestellte Biosuspension gemäß der Fig. 3 dem Hydrolysebehälter 1 eines Kombinierten-Hydrolyse-Fermentations-Apparates, der in der Anlage für die energetische Verwertung von Biomasse mit einer Feuerungswärmeleistung von 3,8 MW in zweifacher Ausführung zur Verfügung steht, zugeführt.

Dazu sind nachträglich in zwei bestehenden Endlagern 10 jeweils Hydrolysebehälter 1 als geschlossene zylinderförmige Konstruktionen aus Edelstahl mit 6,5 m Durchmesser und 12 m Bauhöhe zentrisch positioniert worden. Das Nutzvolumen 19 der bisher unabgedeckt betriebenen Behälter aus wasserdichtem Stahlbeton mit einem lichten Durchmesser von 32 m und 7 m Höhe von 4.983 m³ bei einem Nennfüllstand von 6,2 m wird durch den eingeordneten Hydrolysebehälter 1 je Behälterkombination nur um 206 m³ und damit nur unwesentlich verringert. Bei der nachträglichen gasdichten Abdeckung 6 des den Hydrolysebehälter 1 umgebenden Reaktionsraumes spielt der Hydrolysebehälter 1 auch bautechnisch eine beachtliche Rolle. Im Falle des erläuterten Ausführungsbeispiels ist zwischen dem Außenrand der oberen Abdeckung des Hydrolysebehälters 1 und der Oberkante des bisher als Endlager 10 genutzten Betonbehälters eine Doppelmembrangasspeicher-Konstruktion 22 mit kreisringförmigem Grundriss angeordnet worden. Damit wurde aus dem bisherigen Endlager 10 ein beheizter anaerober Reaktionsraum, der bedarfsweise auch noch durch Isolation der Außenwand thermisch stabilisiert werden kann und womit sich die Behandlungsdauer der eingesetzten Biomasse deutlich verlängert.

Mit der Anordnung einer Balkenkonstruktion für die Ablage der Gasmembran zwischen dem Hydrolysebehälter als Mittelstütze und der Oberkante der Außenwand des Betonbehälters wird deren Steifigkeit gegen Verformungen infolge des verstärkten Einflusses von an die luftgestützte Witterungsschutzmembran 23 angreifenden Windkräfte erhöht. Gleichzeitig wird den Forderungen nach Vermeidung der Emission von Klimaschadgasen aus den unbedeckten Endlagern 10 Rechnung getragen. Schließlich steht für die energetische und umweltgerechte Verwertung der im Hydrolysebehälter 1 anfallenden Hydrolysegase 17 in unmittelbarer Nähe der dafür erforderliche anaerobe Reaktionsraum zur Verfügung.

Die zur Umwälzung 7 der Substrate in den die Hydrolysebehälter 1 umgebenden Reaktionsräumen erfolgt mittels höhenverstellbarer Tauchmotorrührwerke, für deren Wartung bei laufendem Betrieb in die Doppelmembrankonstruktion der Behälterabdeckung jeweils zwei Serviceplattformen eingeordnet sind.

### Bezugszeichenliste

1 - Hydrolysebehälter
2 - Behälter für Gärsubstrate
3 - Einrichtungen für die dosierte Luftzuführung
4 - Wandkonstruktion des Hydrolysebehälters
5 - Heizfläche
6 - gasdichte Abdeckung des Behälters für Gärsubstrat
7 - Gasraum des Behälters für Gärsubstrat
8 - Fermenter
9 - Nachfermenter
10 - Endlager
11 - Umwälztechnik des Hydrolysebehälters
12 - Umwälztechnik des Behälters für Gärsubstrat
13 - Tauchhülse
14 - Nutzvolumen des Hydrolysebehälters
15 - Druckluftlanze
16 - Trombe
17 - sauerstoffhaltige Abgase des Hydrolysebehälters
18 - Auslass für Sinkschlamm
19 - Nutzvolumen des Fermenters
20 - Gasraum des umgebenden Reaktionsbehälters
21 - Außenwand des Fermenters
22 - Doppelmembrangasspeicher
23 - Witterungsschutzmembran
24 - Gasüberströmleitung
25 - Rückschlagklappe
26 - Service-Plattform
27 - Steigrohrleitung
28 - Biosuspensionszuführung

## Patentansprüche

1. Kombinierter Hydrolyse-Fermentations-Apparat zur räumlich getrennten Realisierung der Verfahrensstufen Hydrolyse und Methanfermentation in Bioenergieanlagen mittels Nassfermentation, bestehend aus wenigstens einer beheizbaren, mit Umwälzeinrichtungen und bedarfsweise belüftbaren Behälter-Kombination, **dadurch gekennzeichnet,**
**dass** ein korrosionsfest ausgerüsteter Hydrolysebehälter (1) in einem zumindest im Gasraum (20) ebenfalls korrosionsfest ausgerüsteten Behälter für Gärsubstrate (2) zentrisch angeordnet ist,
**dass** die Wandkonstruktion des Hydrolysebehälters (4) wenigstens teilweise als für Heizwasser durchströmbare Heizfläche (5) ausgebildet ist,
**dass** der Hydrolysebehälter (1) abgasseitig mit dem Gasraum (7) des umgebenden Behälters für Gärsubstrat (2) in Verbindung steht und dass für die Belüftung des Hydrolysebehälters (1) Druckluftlanzen (15) und/oder Einrichtungen für die Luftzuführung über die von der Umwälztechnik (11) gebildete Trombe (16) angeordnet sind.

2. Kombinierter Hydrolyse-Fermentations-Apparat nach dem Anspruch 1, **dadurch gekennzeichnet,**
**dass** zumindest der Hydrolysebehälter (1) mit Einrichtungen für die dosierte Luftzuführung (3) in Verbindung steht.

3. Kombinierter Hydrolyse-Fermentations-Apparat nach einem der Ansprüche 1 und 2, **dadurch gekennzeichnet,**
**dass** die Heizfläche (5) des Hydrolysebehälters (1) eine die maximale Medientemperatur im Hydrolysebehälter (1) von bis zu 60 °C gewährleistende Größe aufweist und
**dass** der den Hydrolysebehälter (1) umgebende Behälter für Gärsubstrat (2) ohne spezielle Heizflächen ausgebildet ist.

4. Kombinierter Hydrolyse-Fermentations-Apparat nach einem der Ansprüche 1 bis 3, **dadurch gekennzeichnet,**
**dass** die senkrechten Umfassungswände (4) des Hydrolysebehälters (1) thermisch unisoliert ausgebildet sind und
**dass** die senkrechten Umfassungswände (4) des Hydrolysebehälters (1) die Heizflächen des umgebenden Behälters für das Gärsubstrat (2) sind.

5. Kombinierter Hydrolyse-Fermentations-Apparat nach einem der Ansprüche 1 bis 4, **dadurch gekennzeichnet,**
**dass** der Hydrolysebehälter (1) als Stütze für die gasdichte Abdeckung (6) des Behälters für Gärsubstrat (2) ausgebildet ist und
**dass** der Hydrolysebehälter die Mittelstütze für die Aufnahme des Gurt- oder Balkensystems zur Ablage der Gasspeicherfolie und/oder für die erhöht angeordnete Gasentnahmeleitung ist.

6. Kombinierter Hydrolyse-Fermentations-Apparat nach einem der Ansprüche 1 bis 5, **dadurch gekennzeichnet,**
**dass** der den Hydrolysebehälter (1) umgebende Behälter für Gärsubstrat (2) ein Fermenter (8), ein Nachfermenter (9) oder ein Endlager (10) für Gärsubstrate ist.

7. Kombinierter Hydrolyse-Fermentations-Apparat nach einem der Ansprüche 1 bis 6, **dadurch gekennzeichnet,**
**dass** wenigstens zwei Hydrolyse-Fermentations-Apparate für den wechselseitigen Batchbetrieb des Hydrolysebehälters (1) und/oder des Behälters für Gärsubstrat (2) in Verbindung stehen.

8. Kombinierter Hydrolyse-Fermentations-Apparat nach einem der Ansprüche 1 bis 7, **dadurch gekennzeichnet,**
**dass** der Hydolysebehälter (1) und /oder der umgebende Behälter für Gäsubstrat (2) mit den Wechsel der Umwälztechnik (11, 12) ohne Betriebsunterbrechungen sichernde Tauchhülsen (13) ausgestattet sind.

9. Kombinierter Hydrolyse-Fermentations-Apparat nach einem der Ansprüche 1 bis 8, **dadurch gekennzeichnet,**
**dass** das Nutzvolumen (14) des Hydrolysebehälters wenigstens das doppelte Volumen der täglichen Dosismenge für die Bioenergieanlage beträgt.

10. Kombinierter Hydrolyse-Fermentations-Apparat nach einem der Ansprüche 1 bis 9, **dadurch gekennzeichnet,**
**dass** die sauerstoffhaltigen Abgase 17 des Hydrolysebehälters (1) das die Versorgung der im umgebenden Behälter für Gärsubstrat (2) und/oder in einem nachgeschalteten Rieselkörperapparat tätigen Schwefelbakterien für die Schwefelwasserstoffbindung sichernde Reaktionsmittel sind.
